# EUROPÄISCHE PATENTSCHRIFT

(11) **EP 1 419 510 B1**
(45) Veröffentlichungstag und Bekanntmachung des Hinweises auf die Patenterteilung: **24.03.2010**
(21) Anmeldenummer: 02772210.7
(22) Anmeldetag: 26.08.2002
(51) Int. Cl.: H01F 13/00, H01F 41/02

(54) **HARTMAGNETISCHER GEGENSTAND UND VERFAHREN ZUR EINSTELLUNG VON RICHTUNG UND LAGE EINES MAGNETVEKTORS**
MAGNETICALLY HARD OBJECT AND METHOD FOR ADJUSTING THE DIRECTION AND POSITION OF A MAGNETIC VECTOR
OBJET MAGNETIQUE A AIMANTATION PERMANENTE ET PROCEDE DE REGLAGE DE LA DIRECTION ET DE LA POSITION D'UN VECTEUR MAGNETIQUE

(30) Priorität: 24.08.2001 DE 10142934
(43) Veröffentlichungstag der Anmeldung: 19.05.2004
(73) Patentinhaber: Berlin Heart GmbH, 12247 Berlin (DE)
(72) Erfinder: NÜSSER, Peter, 13051 Berlin (DE); KAUFFELDT, Conrad, 12559 Berlin (DE); NEUMANN, Werner, 12247 Berlin (DE); GRAICHEN, Kurt, 13189 Berlin (DE); ARNDT, Andreas, 12489 Berlin (DE)
(74) Vertreter: Pfenning, Meinig & Partner GbR
(86) Internationale Anmeldenummer: PCT/EP2002/009522
(87) Internationale Veröffentlichungsnummer: WO 2003/019587

(56) Entgegenhaltungen:
- US-A- 3 989 777
- PATENT ABSTRACTS OF JAPAN vol. 007, no. 144 (E-183), 23. Juni 1983 (1983-06-23) & JP 58 056401 A (TOKYO SHIBAURA DENKI KK), 4. April 1983 (1983-04-04)

## Beschreibung

Die Erfindung bezieht sich auf einen hartmagnetischen Gegenstand und ein Verfahren zur Einstellung eines Magnetvektors eines hartmagnetischen Gegenstandes gemäß der Oberbegriffe der Ansprüche 1 und 10.

Für die unterschiedlichsten mechanischen, technischen und medizinischen Anwendungen ist die Verwendung von hartmagnetischen Gegenständen bekannt. Unter anderem werden hartmagnetische Gegenstände für Messgeräte und Magnetlager verwendet. Magnetlager, insbesondere für Blutpumpen, die als Herzunterstützungspumpen in den Körper eines Menschen implantiert werden, sind im Gegensatz zu herkömmlichen Lagern verschleißfrei und blutschonend.

Für einige Anwendungen ist eine genauere geometrische Ausrichtung des Magnetvektors eines hartmagnetischen Gegenstandes erforderlich, der über die übliche Nord-Süd-Ausrichtung hinausgeht. Insbesondere in Lagern von Blutpumpen, ist eine exakte Ausrichtung und Korrektur der Richtung und der Lage des Magnetvektors des hartmagnetischen Gegenstandes für die Einhaltung der Lagerspiele des Magnetlagers sehr wichtig.

Allgemein charakteristisch für ein Lager sind die Lagersteifigkeit, das Lagerspiel und das Verschleißverhalten. Bei einem Magnetlager bewegt sich das im Lager geführte Teil vor allem um oder entlang einer eingeprägten magnetischen Achse ohne mechanische Berührung mit anderen Teilen des Gerätes und unabhängig von seiner mechanischen Geometrie. Bei langsamen Bewegungen kann je nach Anwendung auch eine geringere Lagersteifigkeit und Genauigkeit toleriert werden. Vor allem bei schnellen Drehbewegungen und/oder großen bewegten Massen ist durch auftretende Unwucht bzw. die Massenträgheit der geführten Teile eine hohe Lagersteifigkeit innerhalb enger Toleranzen notwendig. Bei einer als künstliches Herzunterstützungssystem eingesetzten axialen Blutpumpe sind hohe Drehzahlen für die Förderleistung bei kleinen Abmessungen erforderlich. Um die Blutbelastung in vertretbaren Grenzen zu halten, ist bei optimierter innerer Pumpengeometrie z. B. ein maximales Spaltmaß zwischen Laufrad und Pumpenrohr von 0,01 mm einzuhalten. Mechanische Lager (z.B. Kugellager) würden die mechanischen Anforderungen leicht erfüllen, jedoch zerstören sie im direkten Blutkontakt zuviel der Blutsubstanz. Werden mechanische Lager für diese Anwendung abgedichtet untergebracht, kann beim derzeitigen Stand der Technik die für diesen Anwendungsfall erforderliche Langzeitdichtheit nicht gewährleistet werden. Außerdem tritt am Übergang zwischen Welle und Abdichtung eine Blutschädigung auf und an den Begrenzungen der Dichtungen besteht eine erhöhte Thrombose-Gefahr. Verschleißfreie freischwebende durch Magnetkräfte gehaltene Pumpenlaufräder minimieren diese Nachteile. Die Lagersteifigkeit der Magnetlager des Laufrades beinhaltet aber ein begrenztes Lagerspiel, das bei begrenztem Bauraum und den für den Pumpdruck erforderlichen hydrodynamischen Belastungen nicht zu unterschreiten ist. Zusätzliche Lagerbelastungen durch Unwucht erweitern dieses Lagerspiel. Um die Unwucht zu minimieren, muß die magnetische Lagerachse möglichst genau mit der geometrischen Lagerachse des angetriebenen Pumpenlaufrades übereinstimmen. Im Anwendungsfall der Blutpumpe müssen zur Begrenzung der Unwucht und zur Einhaltung des Spaltmaßes die Winkelabweichungen der resultierenden Magnetvektoren der Lagermagnete von der geometrischen Rotationsachse unter 0,3° liegen. Die für die Leistungsparameter des Magnetlagers erforderlichen handelsüblichen anisotropen hochkoerzitiven Magnete weisen jedoch gemessene mittlere Abweichungen von bis zu ca. 3° zur Normalen der Polflächen auf, die sich entsprechend der Grundorientierung des Ausgangsmaterials statistisch als Glockenkurvenverteilung um den jeweiligen Mittelwert orientieren. Traditionell aus Standardmaterial in einem Stück hergestellte Magnete ergeben nur eine äußerst geringe Ausbeute an Magneten, welche eine resultierende Magnetvektorabweichung von weniger als 0,3° zur Polnormalen aufweisen.

Grund dafür ist, dass der optimalen oder gewünschten Richtung und Größe des Magnetvektors eines Formteils die statistische Verteilung aller unkompensierten Spinmomente entgegensteht, welche für das magnetische Verhalten verantwortlich sind. Nur bei fehlerfreien Einkristallen gibt es Einbereichsbezirke ohne eine statistische Verteilung. Deren Anwendung kommt jedoch aufgrund ungeeigneter Materialeigenschaften (z. B. zu geringes Energieprodukt) nicht für die Herstellung von Magnetlagern oder anderen technisch relevanten Vorrichtungen in Betracht. Auch bei Werkstoffen mit einer ausgeprägten Anisotropie ist eine deutliche statistische Verteilung der unkompensierten Spinmomente mit einer allerdings stark eingeschränkten Schwankungsbreite vorhanden. Das wirkt sich makroskopisch in statistischen Richtungsschwankungen des resultierenden Magnetvektors innerhalb eines bestimmten Toleranzbereiches aus.
Bei den meisten technischen Applikationen für Permanentmagnete spielt diese Tatsache eine untergeordnete Rolle, da fertigungsbedingte Schwankungen des Magnetvektors um eine gewünschte Nullage tolerierbar sind.
In einigen Applikationen wie z.B. implantierbaren Blutpumpen sind die statistischen Richtungsschwankungen jedoch nachteilig, weil die Verwendung von Permanentmagneten mit einem von der gewünschten Richtung abweichenden Magnetvektor zu einer zu großen Unwucht und damit zu einem zu großen Lagerspiel führt.

Daher ist es für solche Applikationen notwendig, Richtung und Lage eines Magnetvektois eines im wesentlichen hartmagnetischen Gegenstandes im offenen Magnetkreis zu ändern bzw. zu korrigieren. Eine solche Änderung bzw. Korrektur kann auf unterschiedliche Weise erreicht werden.

Eine einfache Möglichkeit besteht in der Verwendung eines isotropen, hartmagnetischen Materials, welches sich in der gewünschten Richtung und Stärke magnetisieren läßt. Für ein solches Verfahren sind derzeit lediglich hartmagnetische Materialien bekannt, die in der maximalen Energiedichte nur den unteren Bereich der technisch zu erzielenden Spitzenwerte abdecken. Materialien mit einer derart geringen Energiedichte können jedoch für Magnetlager der oben beschriebenen Art keine Verwendung finden, weil die geforderten Lagersteifigkeiten nicht erreicht werden.

Sofern höhere Energiedichten benötigt werden, besteht die Möglichkeit, die Amplitude des gewünschten Magnetvektors durch die Wahl des für hohe Energiedichten tauglichen Magnetmaterials und der geometrischen Formgebung zu realisieren. Die Annäherung an die gewünschte Ausrichtung des Magnetvektors zur Geometrie des Bauteils kann dann bei genauer Kenntnis der Lage des resultierenden Magnetisierungsvektors im Ausgangsmagneten durch gezieltes "Schrägschneiden" erreicht werden. Nachteilig sind ein erhöhter Arbeitsaufwand und Materialverbrauch sowie die lediglich innerhalb einer gewissen Abweichung zu erzielende Treffgenauigkeit der Richtung des Magnetvektors.

Weiterhin ist es bekannt, durch gezieltes Ent- oder Aufmagnetisieren von Teilbereichen oder der Gesamtheit eines hartmagnetischen Gegenstandes eine Änderung des Magnetvektors zu realisieren. Dieses Ent- oder Aufmagnetisieren kann durch partielle Felder, asymmetrische Felder, einen veränderten Feldgradienten oder andere Methoden erfolgen. Nachteile dieses Verfahrens sind, dass im Allgemeinen der Energiegehalt des Magneten nicht im vollen Maße ausgenutzt wird. Das trifft auch zu, wenn eine Änderung des Magnetvektors durch das Ausnutzen der Temperaturabhängigkeit der Magneteigenschaften, das heißt durch örtliche oder asymmetrische Erwärmung bzw. Kühlung erzielt wird. darüber hinaus sind aktive Beeinflussungen z.B. durch Kopplung mit entsprechend geformten und gerichteten Spulen, die durch veränderte Ansteuerung variabler in den Korrekturmöglichkeiten sind, bekannt. Diese erfordern jedoch Bauraum und zusätzliche Energie.

Die deutsche Offenlegungsschrift DE 26 07 197 A1 zeigt ein magnettechnisches System mit einem Magnetkreis, der mindestens zwei Magnete und mindestens einen Arbeitsluftspalt enthält. Hierbei ist der die Magnete enthaltende Kreis geschlossen und im Inneren des Kreises ist ein Arbeitsluftspalt aus zwei Weicheisenteilen gebildet, die jeweils zwischen zwei miteinander verbundenen Magneten gleicher Polarität angeordnet sind. Durch den geschlossenen Magnetkreis soll ein starkes Magnetfeld im Arbeitsluftspalt mit geringen äußeren Streuungen erzeugt werden. Aus der Oberfläche von dort gezeigten Polschuhen treten die magnetischen Feldlinien senkrecht aus und folgen dann dem vom Magnetkreis vorgegebenen Feldverlauf. Es werden keinerlei Hinweise auf eine Einstellung der Lage oder Richtung eines resultierenden Magnetvektors dargestellt.

Die Ausführung von anderen hartmagnetischen Gegenständen und Verfahren zum Aufbau von Magnet-Anordnungen sind aus GB 777 315, CH 304 762, US 47 77 464, US 23 20 632, und DE 21 06 227 A bekannt.

In US 2 320 632 wird ein Verfahren zur Verbindung von permanentmagnetischen und weichmagnetischen Teil(en) durch Angießen des Magnetmaterials und Ausführung als einheitlich verbundenes Magnetteil, welches mit einem Schlitz die thermischen Verformungen während des Abkühlvorganges aufnimmt, beschrieben. Der permanentmagnetische Teil befindet sich dabei zwischen weichmagnetischen Polstücken. Dadurch wird eine Beeinflussung der Richtung des Magnetfeldes des permanentmagnetischen Teiles für die vorgenannten technischen Anwendungen nicht möglich.

In US 777315 und CH 304762 wird ein magnetischen Joch als Verbindung zwischen permanentmagnetischen und weichmagnetischen Teilen beschrieben. Das Joch ist Teil eines geschlossenen Magnetkreises, z.B. in einem elektrischen Meßgerät. Der permanentmagnetische Teil befindet sich zwischen weichmagnetischen Polstücken. Dadurch ist eine Beeinflussung der Richtung des Magnetfeldes des permanentmagnetischen Teiles nicht möglich.

Sowohl in DE 2106227 A als auch in DE 2607197 A1 wird ein Luftspaltmagnetsystem beschrieben. Dabei werden in einem magnetischen Kreis permanentmagnetischen Teile in weichmagnetische Teile eingebettet. Eine Beeinflussung der Richtung des Magnetfeldes des permanentmagnetischen Teiles ist nicht beabsichtigt und wäre auch nicht realisierbar, da es durch die anliegenden weichmagnetischen Teile zunichte gemacht würde.

In US4777464 wird ebenfalls ein Luftspaltmagnetsystem mit geschlossenen magnetischen Kreis beschrieben. An ein weichmagnetisches äußeres Joch werden an den Innenseiten zwei einander gegenüberliegende permanentmagnetische Teile gleichpolig einseitig angekoppelt, die aus jeweils zwei Magnetmaterialien zusammengesetzt sind. Auf der einander zugewandten Seite dieser Permanentmagnete wird mit einem erfindungsgemäß gestalteten weichmagnetischen Polschuh der Arbeits-Luftspalt gebildet. Ziel der Anordnung ist es, eine möglichst gleichmäßige Feldverteilung im Arbeits-Luftspalt zu erreichen. Eine Beeinflussung der Richtung des Magnetfeldes des permanentmagnetischen Teiles ist nicht beabsichtigt. Die verwendeten Magnete sollen die gleiche Richtung aufweisen. Jede Richtungsänderung würde am weichmagnetischen Joch und am Polschuh zunichte gemacht werden. Die Amplitude des Magnetvektors wird im klassischen Sinn über eine Veränderung der geometrischen Verhältnisse und Abmessungen der verwendeten unterschiedlichen Magnetsorten erreicht.

Zusammenfassend läßt sich feststellen, dass mit keinem der bekannten Verfahren die Richtung des Magnetfeldes eines hartmagnetischen Teiles beeinflußt werden kann. Der Kern der oben beschrieben Verfahren liegt darin, dass durch den geschlossenen magnetischen Kreis der mit dem vorhandenen Magneten (bzw. Spulen) mögliche Fluß maximal in das Arbeitsgebiet dieser Erfindungen (Luftspalt in US 2 320 632, DE 2 106 227 und DE 2 607 197 und Weichmagnetisches Testobjekt in US 777 315 sowie CH 304 762) eingekoppelt wird, bzw. in US 47 77 464 darauf Wert gelegt wird, dass im Luftspalt des magnetischen Kreises bei bestimmten Werten eine möglichst gleichmäßige Verteilung des Magnetfeldes erreicht wird.

Daher kann mit den bekannten Verfahren eine Ausrichtung des Magnetfeldes nicht vorgenommen werden. Denn der Einfluß an einem hartmagnetischen Gegenstand auf die Richtung des Magnetfeldes wird durch die in den oben genannten Verfahren vorgesehenen Anordnungen mit geschlossenem magnetischen Kreis durch die am hartmagnetischen Gegenstand anliegenden weichmagnetischen Teilstücke sofort aufgehoben. Dadurch wird jede vorherige Änderung oder Einstellung der Richtung des Magnetfeldes zunichte gemacht. Die weichmagnetischen Teile, die nicht in der Sättigung betrieben werden, konzentrieren in der Kontaktfläche zum hartmagnetischen Gegenstand den Magnetfluß in Abhängigkeit vom Permeabilitätsunterschied, jedoch unabhängig von der Richtung. Der Magnetfluß verläuft innerhalb dieser Teile entsprechend dem Differenzgefälle des magnetischen Potentials. Aus den weichmagnetischen Teilen tritt das Feld senkrecht zur Oberfläche an der Stelle aus, an welcher der Permeabilitätssprung zur Umgebung bzw. zum benachbarten Teil stattfindet. Der äußere Verlauf der Feldlinien richtet sich dann nach den vorhandenen äußeren Magnetfeldbedingungen. In den oben beschriebenen Verfahren verlaufen die Feldlinien hauptsächlich innerhalb der erfindungsgemäß vorgegebenen Magnetkreise. Mit diesen Anordnungen ist eine Beeinflussung des Magnetvektors nicht möglich.

Der Erfindung liegt daher die Aufgabe zugrunde, einen hartmagnetischen Gegenstand und ein Verfahren zu dessen Herstellung anzugeben, welcher ohne Beeinflussung durch einen äußeren Magnetkreis einen gewünschten resultierenden magnetischen Vektor aufweist, der sich im Rahmen eines vorgegebenen Toleranzbereiches bewegt, und darüber hinaus der hartmagnetische Gegenstand eine gegenüber dem Stand der Technik höhere maximale Energiedichte besitzen soll.

Die Lösung der Aufgabe wird mit den kennzeichnenden Teilen der Ansprüche 1 und 10 erreicht. Vorteilhafte Weiterbildungen sind in den Unteransprüchen angegeben.

Der Vorteil der Erfindung besteht insbesondere darin, dass die Einstellung bzw. die Korrektur der Richtung und Lage des Magnetvektors eines hauptsächlich hartmagnetischen Gegenstandes durch die Verwendung an sich bekannter Materialien auf einfache Weise erreicht werden kann.

Erfindungsgemäß besteht ein hartmagnetischer Gegenstand, dessen magnetischer Vektor sich im offenen Magnetkreis weitgehend im Rahmen eines vorgegebenen Toleranzbereiches bewegt, aus mindestens einem hartmagnetischen Formteil und mindestens einem weiteren Formelement, die so miteinander kombiniert sind, dass durch Formgebung, Zusammenführung und Ausrichtung der Formteile und Formelemente eine vorgegebene Richtung und Lage des Magnetvektors des hartmagnetischen Gegenstandes an der oder den vorbestimmten Seite (n) erreicht wird. Der Magnetvektor des hartmagnetischen Gegenstandes ist der resultierende Magnetvektor der Magnetvektoren des hartmagnetischen Formteiles und der Formelemente.

Der ausgerichtete hartmagnetische Gegenstand kann auch in geschlossenen Magnetkreisen mit oder ohne Luftspalt verwendet werden. Die Wirkung der ausgerichteten Magnetvektoren darf an der ausgerichteten Seite im Magnetkreis nicht durch benachbarte, sich nicht in der Sättigung befindliche weichmagnetische Teile (z.B. Polschuhe, Joche usw.) vollständig aufgehoben werden.

Gemäß der Ausgestaltung des Anspruches 2 bestehen die weiteren Formelemente aus Materialien, wie aus ferri-, ferro-, antiferromagnetischen, paramagnetischen, superparamagnetischen oder diamagnetischen Materialien.

Gemäß der Ausbildung des Anspruches 3 sind das hartmagnetische Formteil und/oder die Formelemente als rotationssymmetrische Körper ausgestaltet.

Der Anspruch 4 sieht eine nicht rotationssymmetrische Ausbildung des hartmagnetischen Formteiles und der Formelemente vor.

Das hartmagnetische Formteil und die Formelemente können gemäß Anspruch 5 und 6 als kompakte Körper oder als Hohlkörper ausgeführt sein.

Vorteilhäfterweise sind gemäß Anspruch 7 das hartmagnetische Formteil und die Formelemente gegeneinander bewegbar und/oder fixierbar angeordnet.

In einer weiteren Ausgestaltung der Erfindung gemäß Anspruch 8 sind das hartmagnetische Formteil und die Formelemente miteinander fest verbunden. Insbesondere eignet sich dazu ein Verkleben der Teile.

In einer weiteren Ausgestaltung der Erfindung gemäß Anspruch 9 ist das Formelement als Hohlraum im hartmagnetischen Formteil ausgebildet.

Das erfindungsgemäße Verfahren zur Einstellung eines Magnetvektors eines hartmagnetischen Gegenstandes ist **dadurch gekennzeichnet dass** eine Vorabbestimmung der Mag-netvektoren sowohl des hartmagnetischen Formteiles als auch der Formelemente des hartmagnetischen Gegenstands hinsichtlich ihrer Richtung und Lage erfolgt und anschließend durch weitere Formveränderungen in Verbindung mit dem Zusammenfügen und Ausrichten der Formelemente eine vorgegebene Richtung und Lage der resultierenden Magnetvektoren erreicht wird.
Diese erfindungsgemäße zielgerichtete Überlagerung der Magnetvektoren der Formteile führt zu einem resultierenden Magnetvektor im Rahmen des vorgegebenen Toleranzbereiches.

In einer weiteren Ausgestaltung des erfindungsgemäßen Verfahrens gemäß Anspruch 12 erfolgt die Einstellung der Richtung und Lage des resultierenden Magnetvektors eines hartmagnetischen Gegenstandes an den vorgesehenen Seiten durch die Bestimmung und Kontrolle des resultierenden Magnetvektors während oder nach der Zusammenstellung des hartmagnetischen Gegenstandes und der wiederholten zielgerichteten Veränderung dieser Zusammenstellung entsprechend der resultierenden Veränderungen des Magnetvektors.

Die erfindungsgemäße zielgerichtete Überlagerung des Magnetvektors eines hartmagnetischen Formteils mit den Magnetvektoren von mehreren Formelementen ist mit ferro-, ferri-, antiferro-, dia-, para- oder superparamagnetischen Materialien möglich. Die Formteile können dabei nebeneinander, übereinander, vollständig oder teilweise ineinander, vollflächig gegeneinander, teilflächig gegeneinander, symmetrisch oder unsymmetrisch zur verändernden Achse, gegeneinander verdreht, rotationssymmetrisch und gegeneinander verdreht, rotationssymmetrisch und schräg, schräg oder gerade in Verbindung mit Abstandseffekten eingesetzt, mit oder ohne Veränderung der Stärke und Richtung durch Zwischenscheiben, schräg geschnitten, keilförmig oder beliebig gegeneinander gesetzt, mit Formschluss, verklebt, oder anderweitig fixiert angeordnet werden.

Der erfindungsgemäße hartmagnetische Gegenstand wird insbesondere als Bestandteil eines Magnetlagers verwendet.

Die Erfindung wird anhand von Zeichnungen und Ausführungsbeispielen näher erläutert. Es zeigen
- Fig. 1: eine rotationsmäßige Ausbildung eines hartmagnetischen Formteiles,
- Fig. 2: eine rotationssymmetrische Ausbildung eines weiteren hartmagnetischen Formelementes,
- Fig. 3: einen erfindungsgemäßen hartmagnetischen Gegenstand, bestehend aus einem hartmagnetischen Formteil und einem Formelement,
- Fig. 4a: einen erfindungsgemäßen hartmagnetischen Gegenstand, bestehend aus einem hartmagnetischen Formteil und zwei Formelementen,
- Fig. 4b: eine Draufsicht auf einen erfindungsgemäßen hartmagnetischen Gegenstand, bestehend aus einem hartmagnetischen Formteil und zwei Formelementen,
- Fig. 5: einen erfindungsgemäßen hartmagnetischen Gegenstand, bestehend aus einem hartmagnetischen Formteil und zwei Formelementen,
- Fig. 6 und 7: einen erfindungsgemäßen hartmagnetischen Gegenstand, bestehend aus einem hartmagnetischen Formteil, einem Formelement und einem weichmagnetischen Formelement und
- Fig. 8 bis 17: weitere Ausgestaltungen hartmagnetischer Gegenstände.

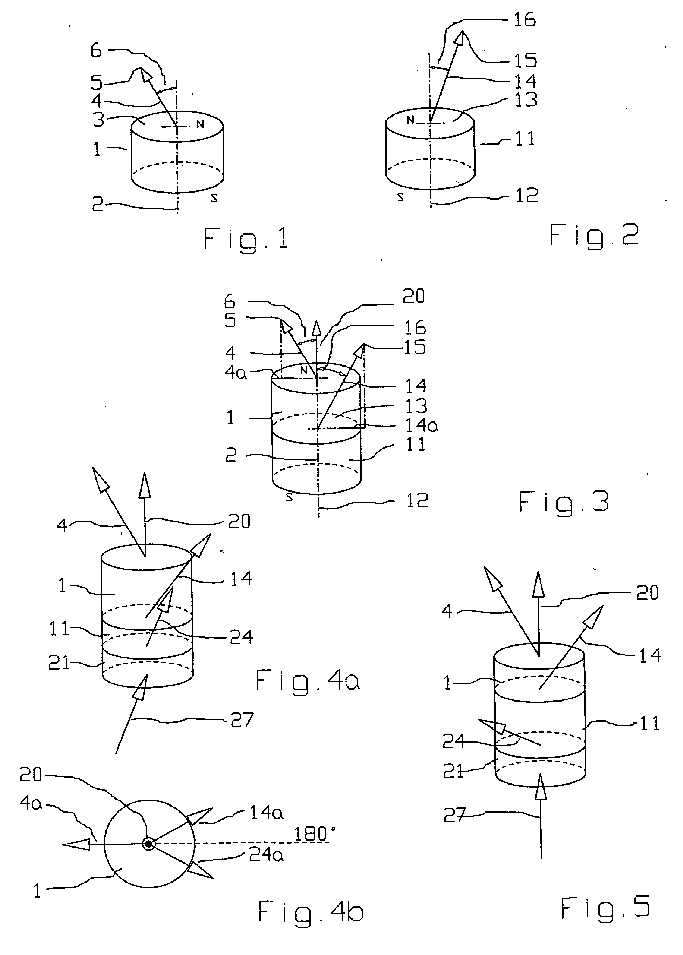

Fig. 1 und Fig. 2 zeigen ein hartmagnetisches Formteil 1 und ein Formelement 11, welche axial magnetisiert Formteile und rotationssymmetrisch geformt sind. Eine Symmetrieachse 2 des Formteiles 1 und eine Symmetrieachse 12 des Formelementes 11 stehen senkrecht auf den Stirnflächen 3 und 13, die hier beispielhaft als Nordpol ausgebildet sind.

Fig. 3 zeigt einen erfindungsgemäßen rotationssymmetrischen hartmagnetischen Gegenstand, der aus einem hartmagnetischen Formteil 1 und einem Formelement 11 besteht. Das Formteil 1 weist einen Magnetvektor 4 mit einer Intensität 5 (Länge des Vektors) auf. Das Formelement 11 weist einen Magnetvektor 14 mit einer Intensität 15 auf. Die Winkel 6 und 16 symbolisieren die Fehlstellung der Magnetvektoren 4 und 14 zur gewünschten Lage (hier zur Symmetrieachse). Durch die Überlagerung der Magnetvektoren 4 und 14 entsteht ein resultierender Magnetvektor 20, wobei die Überlagerung der Magnetvektoren 4, 14 beispielsweise durch Drehung des Formteiles 1 oder des Formelementes 11 eingestellt werden kann, um den vorgegebenen Toleranzbereich des Magnetvektors 20 einzustellen.

Die Kompensation der Winkel 6 und 16 in Fig. 3 erfolgt nicht einfach mittels bekannter grafischer Addition. Deshalb sind die Intensitäten 5 und 15 sowie die Winkel 6 und 16 in den Figuren 1 bis 3 nur zur Demonstration des erfindungsgemäßen Verfahrens zu verstehen. In Fig. 3 sind nur die Magnetvektoren der oberen Nordseite eingetragen. Die Resultierende der Südseite liegt außerhalb der Symmetrieachse.

In Fig. 3 soll die Ausrichtung des resultierenden Magnetvektors 20 mit der Symmetrieachse 2 und 12 zusammenfallen. Die zielgerichtete Ausrichtung des Magnetvektors 20 erfordert eine genaue Messung der Lage und Amplitude der Magnetvektoren der Teile. Beispielsweise wird zunächst die genaue Lage des Magnetvektors 4 des Formteils 1 gemessen. Die Projektion 4a des Magnetvektors 4 auf die Nordpolseite wird gekennzeichnet, beispielsweise auf der Stirnfläche 13 mit einem Strich. Die senkrecht zur Polnormalen wirkende Komponente des Magnetvektors 4 von Formteil 1 ist zu kompensieren durch eine in der Oberfläche von Formteil 1 wirkende, in der Amplitude identische, aber um 180° versetzte Komponente des Magnetvektors 14 von Formelement 11. Zu beachten ist, daß es nicht die senkrecht zur Polnormalen wirkende gemessene Komponente des Magnetvektors 14 von Formelement 11 ist, sondern dass es die Amplitude der Komponente des Magnetvektors 14 ist, die nach dem Zusammenbau des magnetischen Gegenstandes in der Oberfläche von Formteil 1 wirkt. D.h. eine unter messtechnisch vergleichbaren Bedingungen wie Formteil 1 gemessene Größe der Komponente senkrecht zur Polnormalen des Magnetvektors 14 von Formelement 11 muss um einen vom Material und von der geometrischen Abmessung abhängigen Paarungsfaktor größer sein als die Komponente im Formteil 1. Die Größe der Amplitude des Magnetvektors direkt in Normalenrichtung des Poles ist dagegen für die Kompensation der Richtung nicht relevant, sondern nur für die Größe der resultierenden Amplitude des hartmagnetischen Gegenstandes. Der Paarungsfaktor ist zu berechnen oder durch Versuche mit nachfolgender Ergebniskontrolle anzunähern. Das Formelement 11, das den für die Kompensation zulässigen Winkelwert vorweist, wird beispielsweise aus einer Anzahl vermessener und analog Formteil 1 mit der Auslenkungsrichtung markierter Magnete herausgesucht. Der Winkelwert von Formelement 11 darf nur im Bereich einer zulässigen Schwankungsbreite von einem aus der Multiplikation von Abweichungswinkel Formteil 1 mal dimensionslosen Paarungsfaktor errechneten Winkelwert abweichen. Um in diesem Beispiel den resultierenden Magnetvektor in Richtung der Rotationssymmetrieachse auszurichten, wird das ausgewählte Formteil 11, mit einer um 180° in Achsrichtung gegenüber dem Formelement 1 verdrehten Markierung, welche die Projektion 14a darstellt, mit dem Nordpol der Stirnfläche 13 mittig an den Südpol von Formteil 1 positioniert.

Fig. 4a zeigt einen hartmagnetischen Gegenstand, welcher aus dem hartmagnetischen Formteil 1 und den Formelementen 11, 21 zusammengesetzt ist. Hierbei erzeugen die zwei unteren Formelemente 11 und 21 die Kompensation der Winkelabweichung des Magnetvektors 4 von Formteil 1 von der gewünschten Lage. Das Formelement 21 korreliert mit dem Magnetvektor 24. Der untere resultierende Magnetvektor 27 ist nicht zur Rotationsachse parallel. Die Projektion der Magnetvektoren 4a, 14a, 24a in die Ebene der Stirnfläche 3 vom hartmagnetischen Formteil 1 ist in Fig. 4b als Draufsicht auf das hartmagnetische Formteil 1 und die Formelemente 11, 21 dargestellt und verdeutlicht das Prinzip der Magnetvektorausrichtung. Die Länge der Pfeile entspricht der in der Ebene 3 vom hartmagnetischen Formteil 1 senkrecht zur Rotationsachse wirkenden Komponenten der Magnetvektoren 4, 14, 24 der einzelnen Formteile (4a als Komponente von Magnetvektor 4 von Formteil 1; 14a als Komponente von Magnetvektor 14 von Formelement 11; 24a als Komponente von Magnetvektor 24 von Formelement 21). Die Komponenten 14a und 24a müssen gleich groß und mindestens die halbe Amplitude der Komponente 4a aufweisen, dann kann durch gegenseitiges Verdrehen der Teile 11 und 21 um die Rotationsachse die kompensierende Größe gegenüber der Auslenkung des Magnetvektors von Formteil 1 zwischen Null und der maximal möglichen Kraft eingestellt und der zu kompensierenden Größe angepaßt werden. Der untere resultierende Magnetvektor 27 liegt in diesem Ausführungsbeispiel außerhalb der Rotationsachse.

Fig. 5 zeigt einen hartmagnetischen Gegenstand, welcher aus in einer Rotationsachse angeordneten hartmagnetischem Formteil 1 und den Formelementen 11, 21 besteht. In dieser Anordnung der Vektoren 4, 14, 24 wird der obere resultierende Magnetvektor 20 als auch der untere resultierende Magnetvektor 27 zur Rotationsachse ausgerichtet. Das mittlere Formelement 11 mit seinem Magnetvektor 14 erzeugt durch seine Lage und Richtung den Ausgleich für die Vektoren 4 und 24.

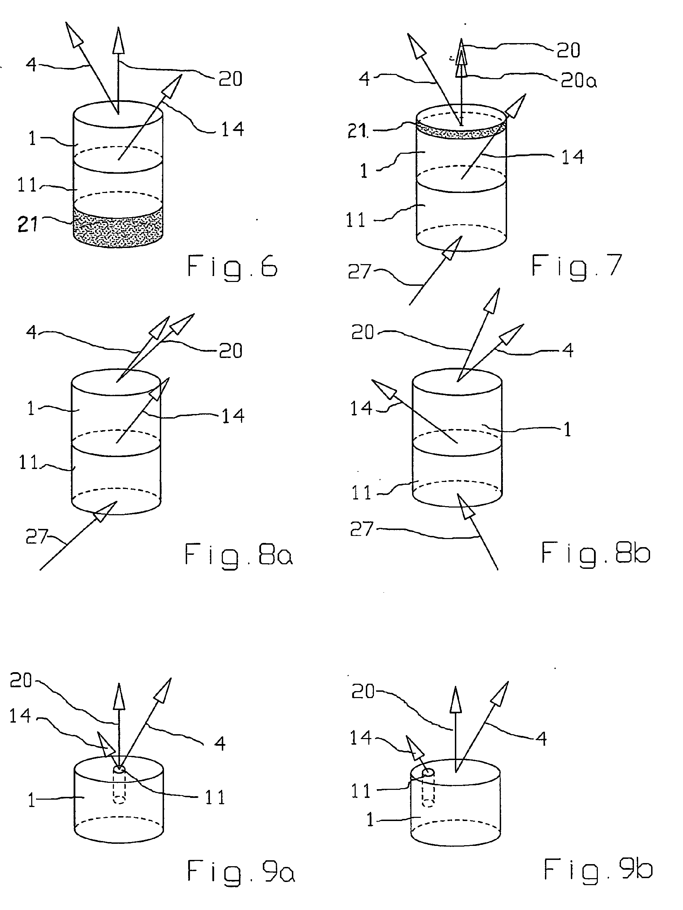

Fig. 6 zeigt einen hartmagnetischen Gegenstand, welcher aus in der Rotationsachse angeordnetem Formteil 1, dem Formelement 11 und einem weichmagnetischen Formelement 21 besteht. In dieser Anordnung der Vektoren 4, 14 wird der obere resultierende Magnetvektor 20 zur Rotationsachse ausgerichtet. Der mittlere Teil 11 mit seinem Magnetvektor 14 erzeugt durch seine Lage und Richtung den Ausgleich für den Vektor 4. Das untere weichmagnetische Formelement 21 befindet sich nicht in der Sättigung und neutralisiert die eingeprägte Winkellage. Die Feldlinien treten aus der Oberfläche des weichmagnetischen Formelementes 21 in Normalen-Richtung aus und folgen dann dem äußeren magnetischen Feld.

Fig. 7 zeigt einen hartmagnetischen Gegenstand, welcher aus einem weichmagnetischen Formelement 21 und in der Rotationsachse angeordnetem Formteil 1 und dem Formelement 11 besteht. In dieser Anordnung der Vektoren 4, 14 wird der obere resultierende Magnetvektor 20 zur Rotationsachse ausgerichtet. Der mittlere Teil 1 mit seinem Magnetvektor 4 erzeugt durch seine Lage und Richtung den Ausgleich für den Vektor 14 des Formelements 11. Das weichmagnetische Formelement 21 gleicht geringfügige flächige Schwankungen der Magnetvektoramplitude des hartmagnetischen Formteiles 1 und des Formelementes 11 aus. Wenn sich das Formelement 21 in der Sättigung befindet, wird die sich vorher aus der Überlagerung der Magnetvektoren 4 und 14 ergebende Richtung des Magnetvektors 20 (resultierender Vektor ohne Formelement 21) nicht neutralisiert. Die Richtung des resultierenden Magnetvektors 20a mit dem Formelement 21 behält in etwa die Richtung des Magnetvektors 20 bei und weist dabei eine veränderte Amplitude auf.

Die Fig. 8a und 8b zeigen hartmagnetische Gegenstände bestehend aus in der Rotationsachse angeordnetem Formteil 1 und dem Formelement 11. In dieser Anordnung der Vektoren 4, 14 wird der obere resultierende Magnetvektor 20 und der untere resultierende Magnetvektor 27 außerhalb der Symmetrieachse ausgerichtet.

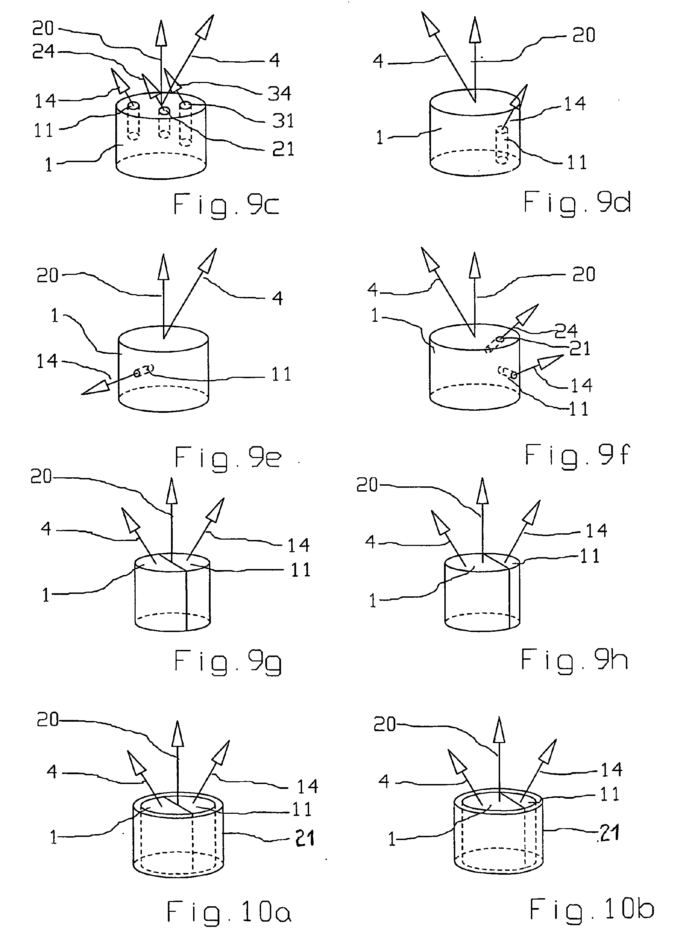

Die Fig. 9a bis 9f zeigen Beispiele von rotationssymmetrischen hartmagnetischen Gegenständen, die aus einem hartmagnetischen Formteil 1 und einem oder mehreren Formelementen 11, 21, 31 zusammengesetzt sind, wobei die Formelemente 11, 21, 31 als Hohlräume im hartmagnetischen Formteil 1 ausgebildet sind. Die Ausrichtung der Vektoren wird dabei beispielsweise so dargestellt, daß der obere resultierende Magnetvektor 20 mit der Symmetrieachse zusammenfällt.

Die Fig. 9g und 9h zeigen hartmagnetische Gegenstände, welche aus einem Formteil 1 und einem Formelement 11 bestehen.

Die Fig. 10a und 10b zeigen Beispiele hartmagnetischer Gegenstände entsprechend Fig. 9g und Fig. 9h, die zur Aufnahme der abstoßenden Kräfte mit einem unmagnetischen Formelement 21 (z.B. Aluminium) ummantelt sind.

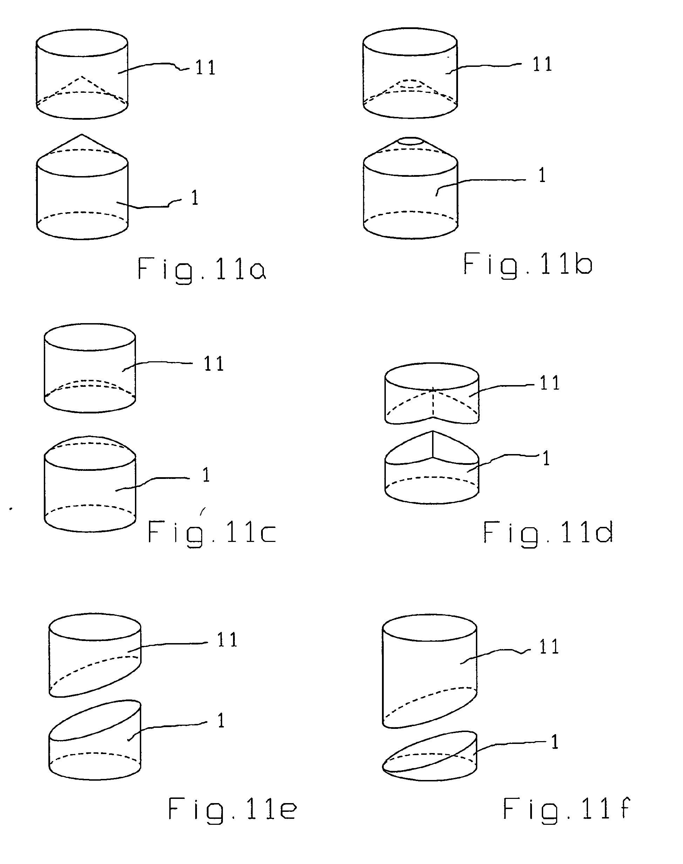

Die Fig. 11a bis 11f zeigen Beispiele von rotationssymmetrischen hartmagnetischen Gegenständen, die aus je einem hartmagnetischen Formteil 1 und einem Formelement 11 zusammengesetzt sind. Diese Figuren veranschaulichen weitere Beispiele von Fügestellen.

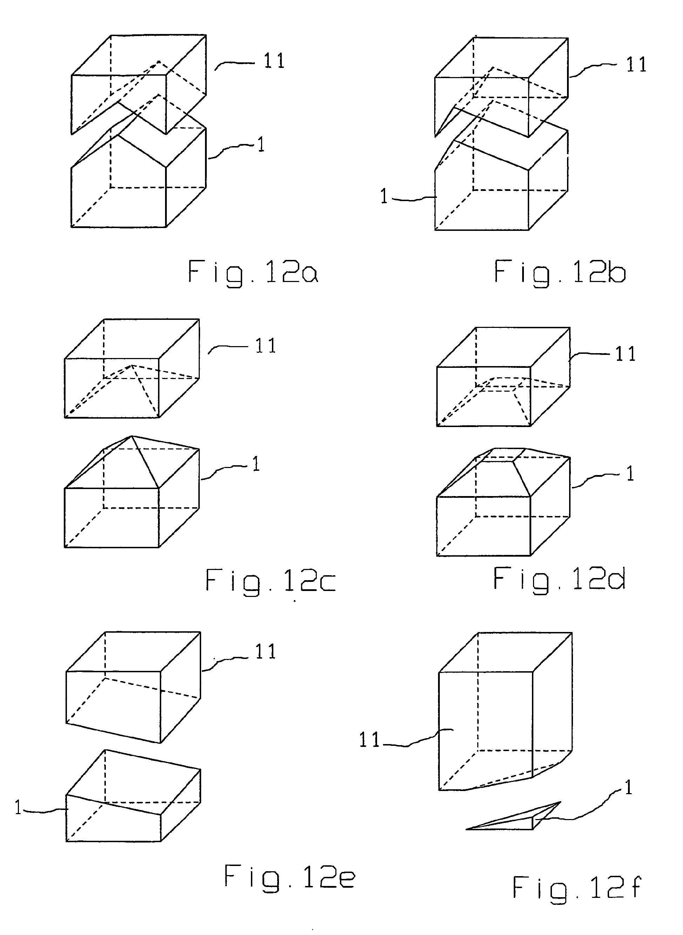

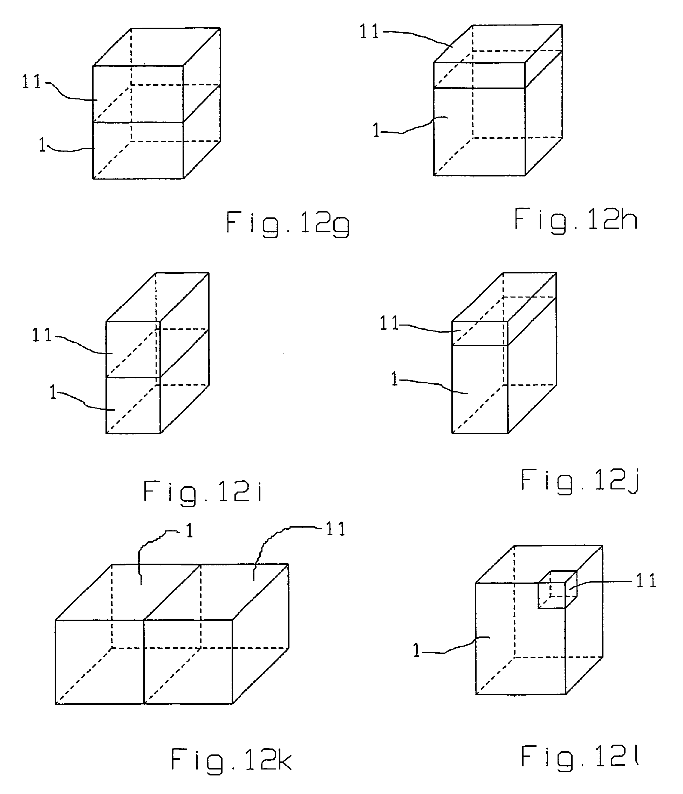

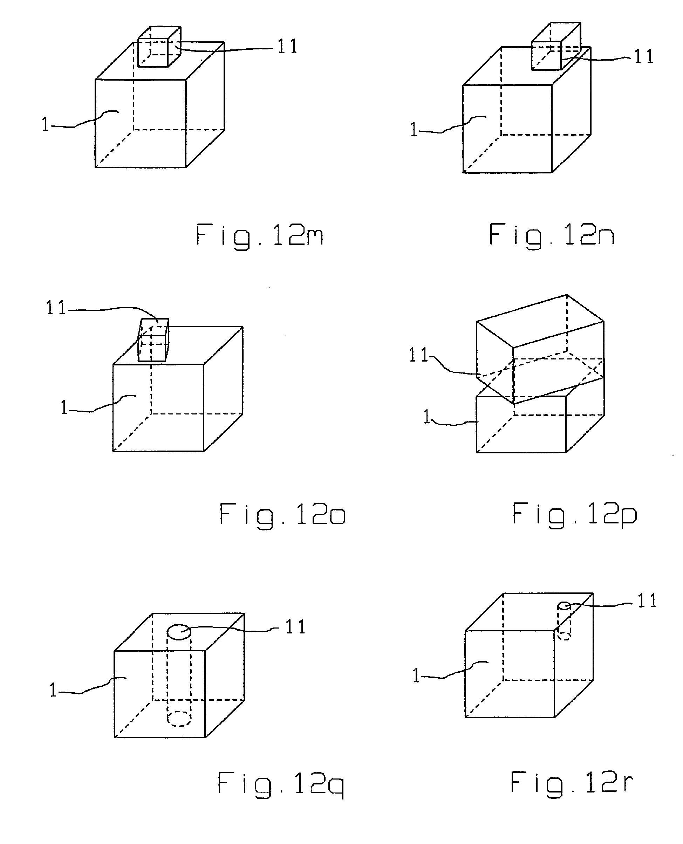

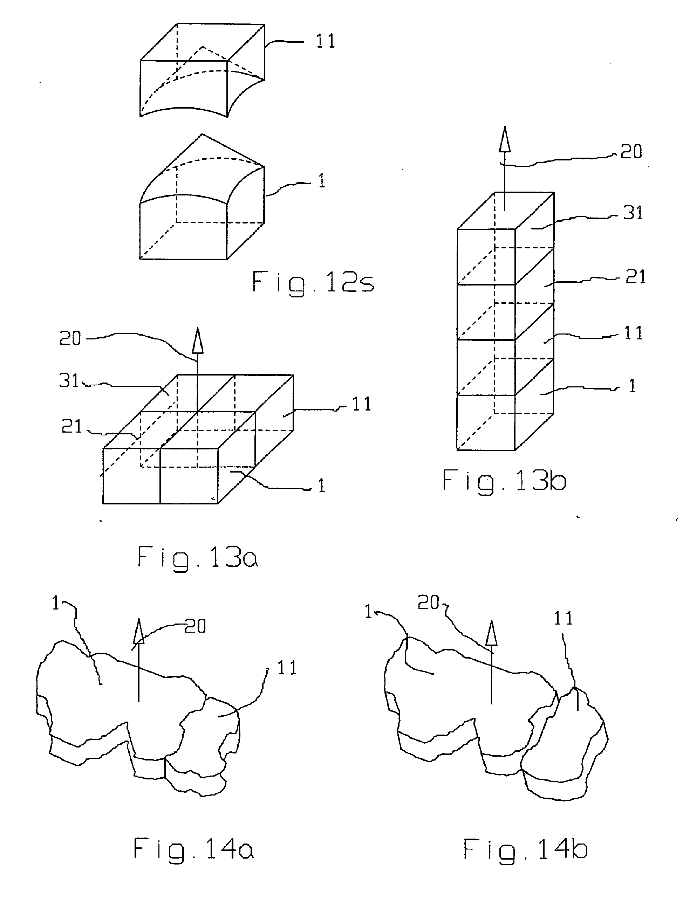

Die Fig. 12a bis 12s zeigen Beispiele von rechteckigen hartmagnetischen Gegenständen, die aus je einem hartmagnetischen Formteil 1 und einem Formelement 11 zusammengesetzt sind. Diese Zeichnungen veranschaulichen weitere Beispiele für die Zusammensetzung der Komponenten.

Die Fig. 13a bis 13b zeigen zwei Beispiele von rechteckigen hartmagnetischen Gegenständen, die aus einem hartmagnetischen Formteil und mehreren Formelementen 11, 21, 31 zusammengesetzt sind, um einen in der Lage und Richtung gewünschten resultierenden Magnetvektor 20 zu erhalten.

Die Fig. 14a und 14b zeigen Beispiele von beliebig geformten hartmagnetischen Gegenständen, die in den Beispielen aus je einem hartmagnetischen Formteil 1 und einem Formelement 11 zusammengesetzt sind. In den. Beispielen wird im magnetischen Schwerpunkt der resultierende Magnetvektor 20 in Normalenrichtung ausgerichtet. Das hartmagnetische Formteil 1 und das Formelement 11 können auch (anders als im Beispiel gezeigt) beliebig geformte Ober- und Unterseiten besitzen. Diese Formteile können in beliebiger Lage formschlüssig oder auch nicht mit der Oberfläche zusammenpassend aneinander oder auch in gewisser Entfernung voneinander gepaart werden (z.B. im Klebeverbund oder im Verguß o.ä.), so daß durch die Addition der Magnetvektoren der Formteile die Lage und Richtung des resultierenden Magnetvektors 20 erreicht wird.

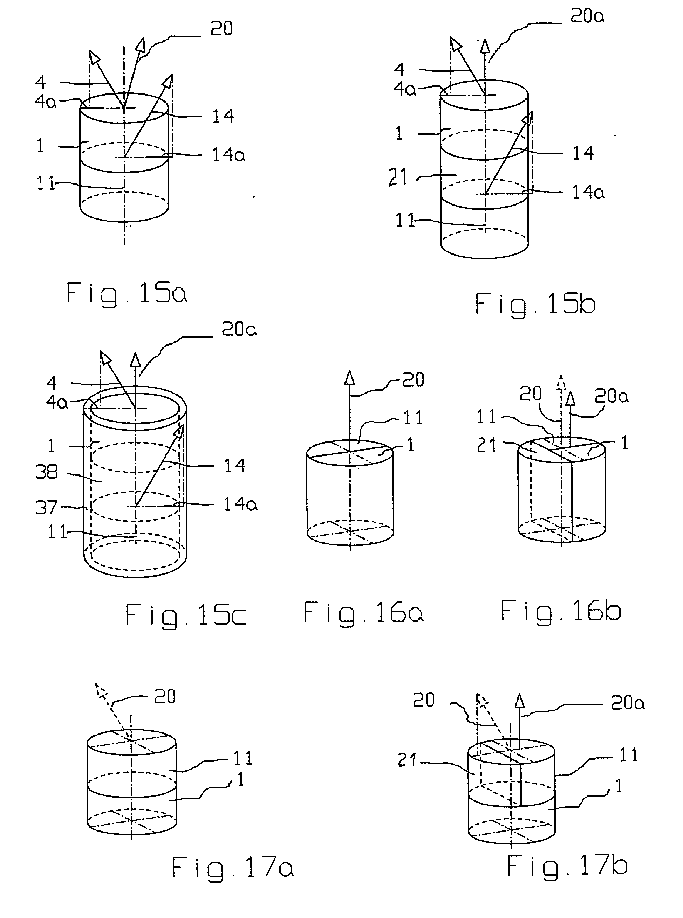

Die Fig. 15b zeigt ein Beispiel eines hartmagnetischen Gegenstandes, der aus einem hartmagnetischen Formteil 1 und einem Formelement 11 und einem "unmagnetischen" (z.B. para- oder diamagnetischen) Formelement 21 zusammengesetzt ist und dessen oberer resultierender Magnetvektor 20 mit der Rotationsachse zusammenfallen soll. In Fig. 15a wird zur Verdeutlichung der fiktive Ausgangszustand gezeigt, in dem das hartmagnetische Formteil 1 und das Formelement 11 ohne Abstand in gleicher Ausrichtung wie in Fig. 15b und Fig. 15c direkt aufeinander gesetzt werden. Das hartmagnetische Formteil 1 und das Formelement 11 ergeben in dieser fiktiven Ausgangslage einen nach oberen gerichteten resultierenden Magnetisierungsvektors 20, der nicht mit der Achse der Rotationssymmetrie zusammenfällt. Ist in dieser Ausgangslage die in der Oberfläche von Formteil 1 senkrecht zur Polnormalen wirkende Vektorkomponente von Formelement 11 größer als die von Formteil 1, so kann mit einer Abstandsvergrößerung die gewünschte Richtungskorrektur erfolgen. In den Fig. 15b und 15c wird mit dem hartmagnetischen Formteil 1 und dem Formelement 11 in einer polgleichen, jedoch um 180° versetzten Ausrichtung der senkrecht zur Polnormalen wirkende Vektorkomponente des Formteiles 1 und des Formelementes 11 der resultierende Magnetvektor 20a in die im Beispiel gewünschte Normalenausrichtung des Magnetvektors korrigiert, und zwar durch eine Abstandsvergrößerung mit einem "unmagnetischen" Formelement 21 bzw. einem Leerraum 38 (Vakuum, gasförmige oder flüssige Füllung), die durch einen Distanzhalter 37 fixiert sind.

Fig. 16b zeigt ein Beispiel eines hartmagnetischen Gegenstandes, der aus dem hartmagnetischen Formteil 1 und dem Formelement 11 und einem unmagnetischen (para- oder diamagnetisches Material) Formelement 21 zusammengesetzt ist. In Fig. 16a wird zur Veranschaulichung der fiktive Ausgangszustand für den hartmagnetischen Gegenstand entsprechend Fig. 16b gezeigt. Das Ausgangsformteil 1 und das Ausgangsformelement 11 würden in Fig. 16a in der nach oben gerichteten Magnetisierung eine Ausrichtung des resultierenden Magnetisierungsvektors 20 ergeben, der mit der Rotationsachse zusammenfällt. In Fig. 16b entfallen die durch das "unmagnetische" Formelement 21 ausgefüllten Anteile des Formteiles 1 und des Formelementes 11. Der Beitrag dieser Teile fehlt auch im resultierenden Magnetvektor 20a entsprechend Fig. 16b. Die Amplitude des resultierenden Magnetvektors 20a verringert sich entsprechend der fehlenden Anteile und die Lage rückt in den neuen magnetischen Schwerpunkt außerhalb der Rotationsachse. Die Richtung zur Polebene bleibt im großen und ganzen bestehen. Bei zusammengesetzten Formteilen kann sich auch die Richtung ändern.

Fig. 17b zeigt ein weiteres Beispiel eines hartmagnetischen Gegenstandes, der aus dem hartmagnetischen Formteil 1 und dem Formelement 11 und einem unmagnetischen (para- oder diamagnetisches Material) Formelement 21 zusammengesetzt ist. In Fig. 17a wird zur Veranschaulichung der fiktive Ausgangszustand für den hartmagnetischen Gegenstand entsprechend Fig. 17b gezeigt. Das Ausgangsformteil 1 und das Ausgangsformelement 11 würden in Fig. 17a in der nach oben gerichteten Magnetisierung eine Ausrichtung des resultierenden Magnetisierungsvektors 20 ergeben, welcher mittig liegt, aber nicht mit der Rotationsachse zusammenfällt. In Fig. 17b entfällt der durch das "unmagnetische" Formelement 21 ausgefüllte Anteil des Formteils 1. Der Beitrag dieses Formteils fehlt auch im resultierenden Magnetvektor 20a entsprechend Fig. 17b. Die Amplitude des resultierenden Magnetvektors 20a verringert sich entsprechend dem fehlenden Anteil, die Lage rückt in den neuen magnetischen Schwerpunkt außerhalb der Rotationsachse und die Richtung verändert sich im Beispiel in Richtung der Polnormalen.

Die einzelnen Teile in den Bildern Fig. 1 bis Fig. 17 können auch aus mehreren Teilen bestehen.

Die Erfindung ist nicht beschränkt auf das hier dargestellte Ausführungsbeispiel. Vielmehr ist es möglich, durch Kombination und Modifikation der genannten Mittel und Merkmale weitere Ausführungsvarianten zu realisieren, ohne den Rahmen der Erfindung zu verlassen.

### Bezugszeichenliste

- 1: hartmagnetisches Formteil
- 2: Symmetrieachse
- 3: Stirnfläche
- 4: oberer Magnetvektor
- 4a: Projektion des Vektors 4
- 5: Intensität
- 6: Winkel zwischen Magnetvektor und Symmetrieachse
- 11: Formelement
- 12: Symmetrieachse
- 13: Stirnfläche
- 14: oberer Magnetvektor
- 14a: Projektion des Vektors 14
- 15: Intensität
- 16: Winkel zwischen Magnetvektor und Symmetrieachse
- 20: oberer resultierender Magnetvektor
- 20a: oberer resultierender Magnetvektor
- 21: Formelement
- 24: oberer Magnetvektor des Formteils 21
- 24a: Projektion des Vektors 24

- 27: unterer resultierender Magnetvektor

- 31: Formelement

- 34: oberer Magnetvektor
- 37: Distanzhalter
- 38: Leerraum (z.B. Luft)

## Patentansprüche

1. Hartmagnetischer Gegenstand, bestehend aus einzelnen zusammengesetzten Teilen, wobei mindestens ein hartmagnetisches Formteil (1) mit mindestens einem Formelement (11, 21, 31) miteinander kombiniert sind, **dadurch gekennzeichnet, dass** im offenen Magnetkreis und ohne Beeinflussung durch einen äußeren Magnetkreis durch Formgebung, Zusammenführung und Ausrichtung des hartmagnetischen Formteils (1) und der Formelemente (11, 21, 31) eine vorgegebene Richtung und Lage einer Resultierenden (20, 20a) eines Magnetvektors (4) des hartmagnetischen Formteils (1) und der Magnetvektoren (14, 24, 34) der Formelemente (11, 21, 31) an den vorgesehenen Seiten erreicht wird.

2. Hartmagnetischer Gegenstand gemäß Anspruch 1; **dadurch gekennzeichnet, dass** die Formelemente (11, 21, 31) aus ferro-, ferri-, antiferro-, para-, superpara- oder diamagnetischem Material bestehen.

3. Hartmagnetischer Gegenstand nach Anspruch 1 oder 2, **dadurch gekennzeichnet, dass**
das hartmagnetische Formteil (1) und die Formelemente (11, 21, 31) rotationssymmetrische Körper sind.

4. Hartmagnetischer Gegenstand nach Anspruch 1 oder 2, **dadurch gekennzeichnet, dass**
das hartmagnetische Formteil (1) und/oder die Formelemente (11, 21, 31) nicht rotationssymmetrische Körper sind.

5. Hartmagnetischer Gegenstand nach einem der Ansprüche 1 bis 4,
**dadurch gekennzeichnet, dass**
das hartmagnetische Formteil (1) und/oder die Formelemente (11, 21, 31) kompakte Körper sind.

6. Hartmagnetischer Gegenstand nach einem der Ansprüche 1 bis 4,
**dadurch gekennzeichnet, dass**
das hartmagnetische Formteil (1) und/oder die Formelemente (11, 21, 31) Hohlkörper sind.

7. Hartmagnetischer Gegenstand nach einem der Ansprüche 1 bis 6,
**dadurch gekennzeichnet, dass**
das hartmagnetische Formteil (1) und/oder die Formelemente (11, 21, 31) gegeneinander bewegbar und/Oder fixierbar angeordnet sind.

8. Hartmagnetischer Gegenstand nach einem der Ansprüche 1 bis 6,
**dadurch gekennzeichnet, dass**
das hartmagnetische Formteil (1) und/oder die Formelemente (11, 21, 31) miteinander fest verbunden sind.

9. Hartmagnetischer Gegenstand nach einem der Ansprüche 1 bis 8,
**dadurch gekennzeichnet, dass**
das Formelement (11) als Hohlraum im hartmagnetischen Formteil (1) ausgebildet ist.

10. Verfahren zur Einstellung eines Magnetvektors eines hartmagnetischen Gegenstands, bestehend aus einzelnen zusammengesetzten Teilen, wobei mindestens ein hartmagnetisches Formteil mit mindestens einem Formelement mit einander kombiniert sind
**dadurch gekennzeichnet, dass**
eine Vorabbestimmung der Magnetvektoren (4, 14, 24, 34) sowohl des hartmagnetischen Formteils (1) als auch der Formelemente (11, 21, 31) hinsichtlich ihrer Richtung und Lage erfolgt und anschließend im offenen Magnetkreis und ohne Beeinflussung durch einen äußeren Magnetkreis durch Formveränderungen der Formelemente (11, 21, 31) in Verbindung mit dem Zusammenführen und Ausrichten der Formelemente (11, 21, 31) mit dem hartmagnetischen Formteil (1) eine vorgegebene Richtung und Lage der resultierenden Magnetvektoren (20, 20a, 27) an den vorgesehenen Seiten erreicht wird.

11. Verfahren nach Anspruch 10,
**dadurch gekennzeichnet, dass**
die Einstellung der Richtung und Lage des resultierenden Magnetvektors des hartmagnetischen Gegenstandes an den vorgesehenen Seiten erfolgt durch die Bestimmung und Kontrolle des resultierenden Magnetvektors (20) während oder nach der Zusammenstellung des hartmagnetischen Gegenstands und der wiederholten zielgerichteten Veränderung dieser Zusammenstellung entsprechend der resultierenden Veränderungen des Magnetvektors.

12. Verwendung eines hartmagnetischen Gegenstandes gemäß Anspruchs 1 als Bestandteil eines Magnetlagers.

## Claims

1. A hard-magnetic object, consisting of individually put-together parts, wherein at least one hard-magnetic shape part (1) with at least one shape element (11, 21, 21) are combined with one another, **characterised in that** in the open magnetic circuit and without influencing by an external magnetic circuit, a predefined direction and position of a resulting (20, 20a) of a magnetic vector (4) of the hard magnetic shape part (1) and of the magnetic vectors (14, 24, 34) of the shape elements (11, 21, 31) is achieved at the envisaged sides by way of shaping, leading-together and lining the hard-magnetic shape part (1) and the shape elements (11, 21, 31).

2. A hard-magnetic object according to claim 1, **characterised in that** the shape elements (11, 21, 31) are of ferromagnetic, ferrimagnetic, antiferromagnetic, paramagnetic, superparamagnetic or diamagnetic material,

3. A hard-magnetic object according to claim 1 or 2, **characterised in that** the hard-magnetic shape part (1) and the shape elements (11, 21, 31) are rotationally symmetrical bodies.

4. A hard-magnetic object according to claim 1 or 2, **characterised in that** the hard-magnetic shape part (1) and/or the shape elements are not rotationally symmetrical bodies.

5. A hard-magnetic object according to one of the claims 1 to 4, **characterised in that** the hard-magnetic shape part (1) and/or the shape elements (11, 21, 31) are compact bodies.

6. A hard-magnetic object according to one of the claims 1 to 4, **characterised in that** the hard-magnetic shape part (1) and/or the shape elements (11, 21, 31) are hollow bodies.

7. A hard-magnetic object according to one of the claims 1 to 6, **characterised in that** the hard-magnetic shape part (1) and/or the shape elements (11, 21, 31) are arranged in a manner such that they are movable and/or fixable, to one another.

8. A hard-magnetic object according to one of the claims 1 to 6, **characterised in that** the hard-magnetic shape part (1) and/or the shape elements (11, 21, 31) are connected to one another in a fixed manner.

9. A hard-magnetic object according to one of the claims 1 to 8, **characterised in that** the shape element (11) is designed as a cavity in the hard-magnetic shape part (1).

10. A method for setting a magnetic vector of a hard-magnetic object consisting of individually put-together parts, wherein at least one hard-magnetic shape part with at least one shape element are combined with one another, **characterised in that** a prior determination of the magnetic vectors (4, 14, 24, 34) of the hard-magnetic shape part (1) as well as the shape elements (11, 21, 31) with regard to their direction and position is effected, and subsequently in the open magnetic circuit and without influencing by way of an external magnetic circuit, a predefined direction and position of the resulting magnetic vector (20, 20a, 27) at the envisaged sides is achieved by way of shape changes of the shape elements (11, 21, 31) in combination with the leading-together and alignment of the shape elements (11, 21, 31) with the hard-magnetic shape part (1).

11. A method according to claim 10, **characterised in that** the setting of the direction and position of the resulting magnetic vector of the hard-magnetic object at the envisaged sides is effected by way of determining and control of the resulting magnetic vector (20) during or after the composition of the hard-magnetic object and the repeated target-orientated changing of this composition according to the resulting changes of the magnetic vector.

12. The use of a hard-magnetic object according to claim 1 as a component of a magnet bearing.

## Revendications

1. Objet magnétique dur, constitué de pièces individuelles assemblées, dans lequel au moins une pièce moulée magnétique dure (1) est combinée à au moins un élément moulé (11, 21, 31), **caractérisé en ce que**, en circuit magnétique ouvert et sans influence due à un circuit magnétique externe, par façonnage, réunion et alignement de la pièce moulée magnétique dure (1) et des éléments moulés (11, 21, 31), on obtient une direction et une position prédéfinies d'une résultante (20, 20a) d'un vecteur magnétique (4) de la pièce moulée magnétique dure (1) et des vecteurs magnétiques (14, 24, 34) des éléments moulés (11, 21, 31) sur les côtés prévus.

2. Objet magnétique dur selon la revendication 1, **caractérisé en ce que** les éléments moulés (11, 21, 31) sont constitués d'un matériau ferro-, ferri-, antiferro-, para-, superpara- ou diamagnétique.

3. Objet magnétique dur selon la revendication 1 ou 2, **caractérisé en ce que**
la pièce moulée magnétique dure (1) et les éléments moulés (11, 21, 31) sont des corps à symétrie de rotation.

4. Objet magnétique dur selon la revendication 1 ou 2, **caractérisé en ce que**
la pièce moulée magnétique dure (1) et/ou les éléments moulés (11, 21, 31) est ou sont des corps qui ne présentent pas de symétrie de rotation.

5. Objet magnétique dur selon l'une quelconque des revendications 1 à 4, **caractérisé en ce que**
la pièce moulée magnétique dure (1) et/ou les éléments moulés (11, 21, 31) est ou sont des corps compacts.

6. Objet magnétique dur selon l'une quelconque des revendications 1 à 4, **caractérisé en ce que**
la pièce moulée magnétique dure (1) et/ou les éléments moulés (11, 21, 31) est ou sont des corps creux.

7. Objet magnétique dur selon l'une quelconque des revendications 1 à 6, **caractérisé en ce que**
la pièce moulée magnétique dure (1) et/ou les éléments moulés (11, 21, 31) est ou sont aménagés de manière déplaçable et/ou fixable l'un par rapport à l'autre.

8. Objet magnétique dur selon l'une quelconque des revendications 1 à 6, **caractérisé en ce que**
la pièce moulée magnétique dure (1) et/ou les éléments moulés (11, 21, 31) est ou sont raccordés l'un à l'autre de manière fixe.

9. Objet magnétique dur selon l'une quelconque des revendications 1 à 8, **caractérisé en ce que**
l'élément moulé (11) se présente sous la forme d'une cavité dans la pièce moulée magnétique dure (1).

10. Procédé de réglage d'un vecteur magnétique d'un objet magnétique dur, constitué de pièces individuelles assemblées, dans lequel au moins une pièce moulée magnétique dure est combinée à au moins un élément moulé, **caractérisé en ce que**
l'on réalise une détermination préalable des vecteurs magnétiques (4, 14, 24, 34) tant de la pièce moulée magnétique dure (1) que des éléments moulés (11, 21, 31), en matière de direction et de position et, ensuite, en circuit magnétique ouvert et sans influence due à un circuit magnétique externe, par des modifications de forme des éléments moulés (11, 21, 31) en liaison avec la réunion et l'alignement des éléments moulés (11, 21, 31) avec la pièce moulée magnétique dure (1), on obtient une direction et une position prédéfinies des vecteurs magnétiques résultants (20, 20a, 27) sur les côtés prévus.

11. Procédé selon la revendication 10, **caractérisé en ce que**
le réglage de la direction et de la position du vecteur magnétique résultant de l'objet magnétique dur s'effectue sur les côtés prévus en déterminant et en contrôlant le vecteur magnétique résultant (20) pendant ou après l'assemblage de l'objet magnétique dur et la modification ciblée répétée de cet assemblage en fonction des modifications résultantes du vecteur magnétique.

12. Utilisation d'un objet magnétique dur selon la revendication 1 comme composant d'un palier magnétique.
